# EUROPEAN PATENT SPECIFICATION

(11) **EP 4 076 303 B1**
(45) Date of publication and mention of the grant of the patent: **08.10.2025**
(21) Application number: 20903757.1
(22) Date of filing: 18.12.2020
(51) Int. Cl.: F04B 23/02, A61F 9/00, A61M 11/00, F04B 43/04

(54) **VENTED MULTI-DOSE OCULAR FLUID DELIVERY SYSTEM**
BELÜFTETES SYSTEM ZUR VERABREICHUNG VON OKULAREN FLÜSSIGKEITEN IN MEHREREN DOSEN
SYSTÈME D'ADMINISTRATION MULTI-DOSE DE FLUIDE OCULAIRE À ÉVENT

(30) Priority: 20.12.2019 US 201962951903 P; 06.03.2020 US 202016811879; 17.04.2020 US 202063011800 P; 07.07.2020 US 202063049110 P; 08.07.2020 US 202063049582 P
(43) Date of publication of application: 26.10.2022
(73) Proprietor: Bausch + Lomb Ireland Limited, Dublin D24 PPT3 (IE)
(72) Inventor: QUINTANA, Reynaldo, Menlo Park, CA 94025 (US); IVRI, Yehuda, Newport Coast, CA 92657 (US); PALANKER, Daniel, V., Sunnyvale, CA 94087 (US)
(74) Representative: HGF
(86) International application number: PCT/US2020/066169
(87) International publication number: WO 2021/127535

(56) References cited:
- WO-A1-2013/076682
- WO-A1-2013/076682
- US-A1- 2007 102 455
- US-A1- 2007 102 455
- US-A1- 2010 072 301
- US-A1- 2010 147 899
- US-A1- 2019 314 195
- US-A1- 2019 314 195

## Description

### FIELD OF THE INVENTION

This invention relates to topical ocular delivery of ophthalmic medications.

### BACKGROUND

Currently, pharmaceutical fluids are typically delivered to the eye surface using a drop bottle. This method has multiple drawbacks: (1) Patients cannot aim well and often miss the eye; (2) Volume of a drop from a bottle is not well-defined and is too large (on the order of 50 µL) for the tear film on the cornea to absorb it - the tear film can hold no more than about 7 µL; (3) Very often patients blink during the drop delivery, so that part of the drop lands on the eyelid, and the rest is wiped off the cornea.

International patent publication No. WO2013076682A1 discloses a multidose dispenser head, in particular for pharmaceutical products, comprising a dispensing nozzle with shutter housed inside a pressurized chamber wherein the means for housing the shutter comprise an elastically deformable membrane which is arranged so as to sealingly close off said chamber and is connected to the shutter so as to act as an elastic suspension element of the shutter itself.

United States patent publication No. US2019314195A1 discloses methods of administering a liquid formulation of an ophthalmic agent to a topical ocular location of an eye. Aspects of the methods include delivering to the topical ocular location a dose of the liquid formulation that can be wholly accommodated by the tear film of the eye. Devices and kits for carrying out the methods are also disclosed.

United States patent publication No. US2007102455A1 discloses a dispenser for a liquid which is particularly, useful for dispensing liquid medical compositions such as nasal sprays. The dispenser employs at least one, though preferably two, semi-rigid casing halves, defining a liquid reservoir, within which is mounted a bridge member which performs the functions of dip tube, dose metering pump, and outlet orifice.

### SUMMARY

We have developed a device that addresses these problems by (1) delivery of a precise amount of fluid; (2) with a micro-dose that the tear film can hold (<10uL); (3) deliver it within the blink time (~100ms), and (4) using an optical aiming onto the cornea for precise self-administration.

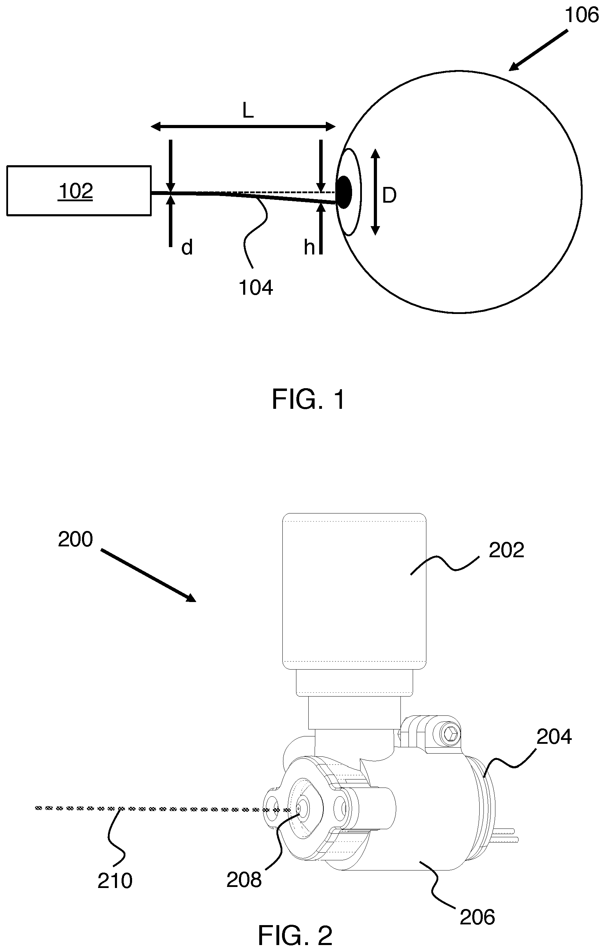

For convenient aiming, the fluid ejector should be placed close to the eye, but not touch the eyelashes or eyebrow. Therefore, with reference to FIG. 1, the device 102 should be in the range of approximately L = 1-10 cm from the eye, or more optimally 2-6 cm. On this figure, 102 is the fluid ejector, 104 is the emitted fluid stream and 106 is the patient's eye.

The cornea is about D=12 mm in diameter, i.e. 6 mm in radius. To ensure that the fluid is delivered approximately to the middle of the cornea, the jet 104 should not deflect under gravity by more than about half the cornea radius, i.e. no more than about h=3 mm. As shown in FIG. 1, vertical deflection h of the projectile ejected horizontally with velocity v over a distance L is: h = g*L/(2v²). To ensure that vertical deflection does not exceed h, horizontal jet velocity should exceed v = L*(g/2h)^{0.5}. For L=5 cm, g= 9.8 m/s², h = 3 mm, we obtain v=2 m/s. For L=5 cm and h = 1 mm, velocity should be about v=3.6 m/s, and for L=10 cm, h=1 mm, velocity v = 7.2 m/s. Therefore overall, jet velocity should be in the range of about 1-10 m/s, and more optimally 2-4 m/s. Velocities much higher than those may cause discomfort to the patient and even damage to the cornea.

The stream of fluid will reach the eye within a few milliseconds from the moment of dispensing (t= L/v, in the range of 1-100ms). As soon as the fluid will touch the cornea, it will trigger the blink reflex, which typically takes about T=100ms. To prevent the drug being blocked by the eyelid, the fluid should be delivered before the eye closure. For the required volume V to be delivered within the time T with the jet velocity v, the jet cross-sectional area should be S=V/(T*v). Since for a round aperture, S = π*d²/4, its diameter d = (4V/(πT*v))^{0.5}. For example, for v = 2 m/s, T=100ms, V = 10 µL, we obtain d = 250 µm. For v = 1 m/s, d = 350 µm, and for v = 7 m/s, d = 130 µm. Therefore, the aperture diameter of the ejector should be in the range of approximately 200 - 600 µm, and more optimally 400-550 µm. Alternatively, several apertures could be used to produce several parallel streams for faster delivery.

Another key attribute of the system is the prevention of microbial ingress to the contained liquid during storage or use. As with any closed system, as liquid is ejected, air should be introduced to replace the ejected volume and thereby balance the pressure (venting). To preclude microbial ingress, the air is introduced via a special inlet preferably having a 0.2um filter. Ideally, the device should operate such that liquid is ejected through the aperture any time it is opened, thereby preventing the air ingress through it.

An exemplary embodiment is an arrangement for storing and discharging liquid droplets having a housing including a chamber for holding liquid therein and having an intake port connected to an ampoule containing pharmaceutical fluid to be dispensed. The chamber includes a dispensing aperture plate which defines a frontal closure to the chamber and includes an aperture opening therein through which the liquid is discharged forwardly of the housing. The chamber further includes a vibrating membrane secured to the housing in pressure transmitting relation with the liquid in the chamber. The vibrating membrane is formed with a needle which protrudes from its center and extends to the aperture in the opposite side of the chamber, said needle closes the aperture to prevent outflow of liquid from the chamber and ingress of bacteria.

An electromagnetic transducer is attached to the housing, and when energized, pulls the membrane rearward against a spring in the chamber. When the electromagnetic transducer is turned off, the spring returns the membrane to its original position with the valve closed. When the electromagnetic transducer is energized with pulsatile or alternating current, the membrane is consequently oscillated, which in turn generates pressure on the liquid. At the correct frequencies, the pressure is sufficient to eject a stream of liquid from the aperture.

Typical range of frequencies is in a range of 10 Hz to 500 Hz, more optimally 50 to 200 Hz. The diameter of the nozzle, velocity of the fluid ejection, and duration of the electromagnetic burst are preferably optimized to deliver the required amount of fluid within the required amount of time, as described above. Preferably the actuation pulse duration is 250 ms or less, and more preferably it is 100 ms or less. Here 'actuation pulse duration' refers to the length of time the electromagnetic transducer is energized so as to pull the needle out of the aperture in a single actuation pulse.

Other kinds of transducer can also be used to drive fluid ejection in this configuration, such as a coin vibration motor.

### BRIEF DESCRIPTION OF THE DRAWINGS

FIG. 1 shows geometry for delivering fluid to an eye of a patient.
FIG. 2 is an exterior view of an exemplary embodiment of the invention.
FIG. 3A is a cross section view of a first embodiment of the invention.
FIGs. 3B-C show operation of the embodiment of FIG. 3A.
FIGs. 4A-B show an example of venting.
FIG. 5 is a cross section view of a second embodiment of the invention.
FIG. 6 shows a conical spring.

### DETAILED DESCRIPTION

FIG. 2 shows a perspective view of a first embodiment of the liquid ejection unit. The fluid ejection unit **200,** is specifically but not exclusively suitable for use in delivering preservative-free pharmaceutical liquid to the surface of the eye. Liquid ejection device **200** comprises a thermoplastic body **206** formed with a liquid chamber and connected to a fluid supply ampule **202.** The ejection unit includes nozzle **208** through which liquid **210** is dispensed as will explained in greater detail below.

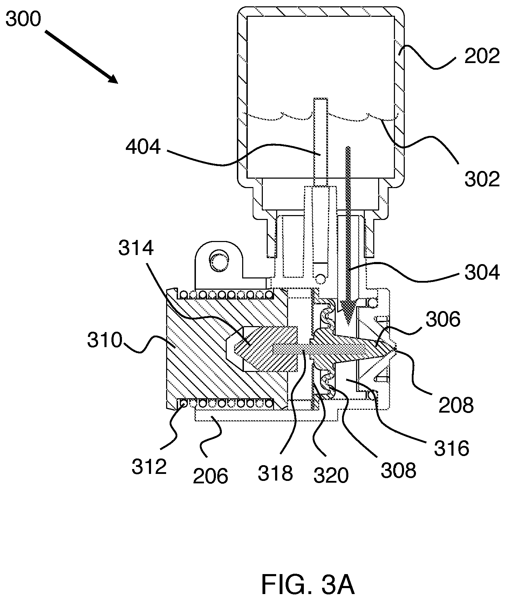

FIG. 3A illustrates a cross-sectional view of the fluid ejection device. As mentioned earlier the fluid ejection device comprises a thermoplastic body **206** defining a chamber **316** connected to the fluid supply ampule **202** containing a fluid **302.** The fluid ejection device includes a nozzle **208** through which liquid is ejected. The device further includes a membrane **308** at the opposite end of the chamber from the nozzle. Membrane **308** includes an integral needle **306** such that they are one component. The needle **306** and the membrane **308** are connected to an electromagnetic transducer **310** via a link member **318.** Upon application of an electrical pulse to the electromagnetic transducer **310,** electric current flows through the coil **312** and a magnetic force is developed which pulls plunger **314** backward against a spring **320.**

On FIG. 3A, **304** shows the direction of fluid flow from ampule **202** to chamber **316.** Vent tube **404** is described in greater detail below.

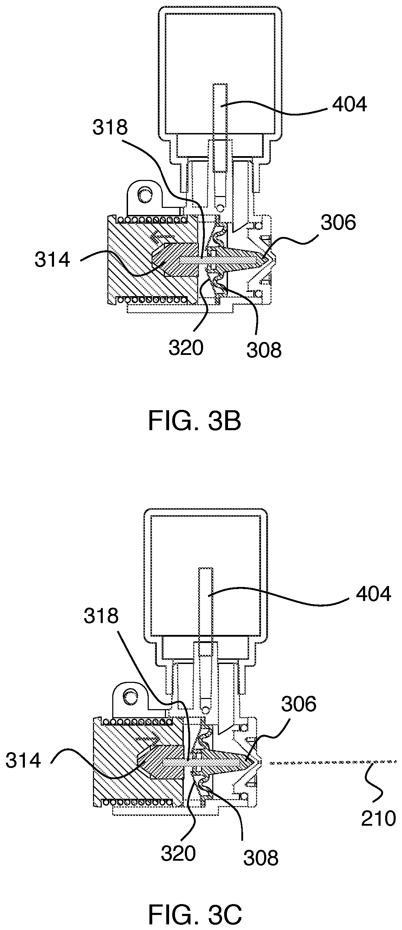

FIG. 3B shows the device of FIG. 3A following application of an electrical pulse to electromagnetic transducer **310.** It can be seen that as a result from the magnetic force, plunger **314** is pulled into the electromagnetic transducer in the direction indicated by the arrow. Membrane **308** is connected to plunger **314** by linkage member **318** and is also pulled back. FIG. 3C shows the situation when the electromagnetic transducer **310** is de-energized. Here the spring **320** pushes the membrane **308** back to its original position, so the valve is closed and the chamber is hermetically sealed and prevents microbial ingress.

When the electromagnetic transducer 310 is energized with a pulsatile or alternating (AC) current, the oscillating membrane generates pressure in liquid resulting in a stream ejected from the aperture. Typically, the operating frequency is from 10 to 500 Hz and more specifically from 50 to 200 Hz. In an embodiment, the membrane **308** is made of silicon having hardness durometer between 50-70 (shore A), and the displacement of plunger **314** is about 200um. Since the flow is produced only in the outward direction, it prevents microbial ingress even when the valve is open.

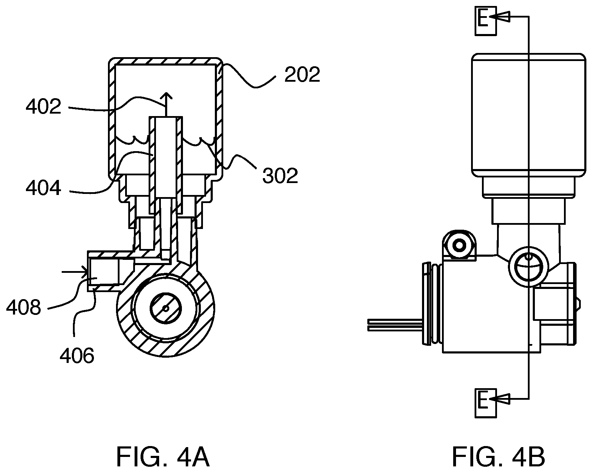

In the example of FIGs. 4A-B, the liquid ejection device includes a venting arrangement to equalize the pressure inside the ampoule **202** with the ambient atmospheric pressure. Here FIG. 4A is a cross section view along line E-E of FIG. 4B.

The venting system of this example includes an air inlet vent tube **404** that is extended beyond the liquid level of fluid **302** in the ampoule **202.** It should be noted that the vent tube **404** is above the liquid level in any orientation of the device of FIGs. 4A-B. Vent tube **404** is connected to venting outlet **406,** which is open to the atmosphere. In one embodiment, a filter **408** is placed in the venting outlet **406** such that the vented air flowing to the ampoule is filtered to prevent penetration of potential airborne contamination, such as microbes. Filter **408** will filter away particles with size > 1 µm (more preferably > 0.5 µm, still more preferably > 0.2 µm). In this way the system can be isolated from microbial contamination, even though air **402** enters the ampule **202** as fluid is emitted.

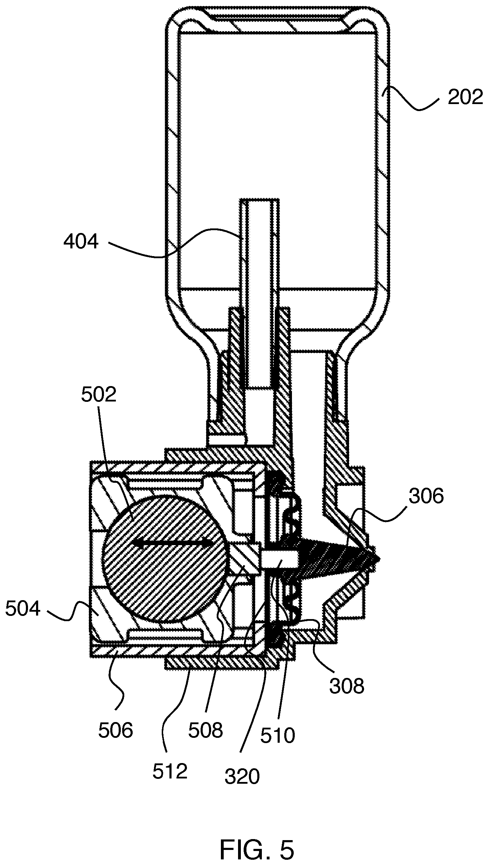

In the preceding examples, diaphragm **308** is driven with a solenoid. In the example of FIG. 5, the diaphragm is driven by using a coin vibration motor. More specifically, **306** is a needle connected to a diaphragm **308** as described above. This assembly is overmolded with a magnetic steel pin **510. 502** is a coin vibrator motor (e.g., JINLONG MACHINERY & ELECTRONICS CO., LTD. model # C1026B002F). **504** is a plastic molded component which holds the motor **502** such that it can slide along rails (i.e., it is a motor holder). **506** is a plastic molded component that provides the above-mentioned rail guides for motor holder **504** to slide within. **510** is a magnetic steel pin which is molded into the membrane/needle assembly (**308/306**). **512** is the housing that holds all the components together.

Needle **306** normally seals the aperture (i.e., the aperture is sealed except when fluid is being emitted), as described in greater detail above. A coin vibration motor has an eccentric weight off its axis of rotation (the axis of rotation is perpendicular to the plane of FIG. 5). Because the weight is off axis, as the motor rotates the unbalanced weight causes the motor to vibrate primarily in the plane of FIG. 5. By placing the coin vibration motor **502** in a plastic motor holder **504** which fits into corresponding rails (in member **506**), the coin vibration motor is constrained so it can only move linearly (e.g., left to right on FIG. 5). As a result of this physical constraint, when the motor rotates, it is only allowed to oscillate left to right, rather than to vibrate in a plane. The coin vibration motor is coupled to the diaphragm **308,** consequently as the motor oscillates left to right, the diaphragm is also vibrated left to right. The ejected fluid stream is generated in the same manner as described above -- i.e., the needle **306** moves back and forth in the aperture to eject the liquid.

In an alternative embodiment, the coin vibration motor **502** can be coupled to the diaphragm via an optional magnet **508.** Magnet **508** is fixed to motor holder **504** which is also affixed to the coin vibration motor **502.** When the magnet **508** is close to the magnetic steel pin **510,** the two latch together and the motor is thereby coupled to the diaphragm. This is an advantageous assembly feature, because the motor can be easily added to the system without the need for tight tolerances and the motor can be added at several different steps of the assembly process.

The example of FIG. 5 includes a disk-shaped spring **320.** The spring is slightly deformed out of plane during assembly which serves to transmit force to needle **306.** This force or load keeps the needle **306** pressed up against the orifice to close the flow path. Without the spring, the force required to push the needle open is very low and the device will readily leak. Additionally, the spring has a spring constant which is important for ensuring the correct frequency and amplitude of oscillation of the needle when the motor is energized. Also important is that without the spring it is only the stiffness of the diaphragm **308** which applies a load to keep the needle **306** in the closed position. The diaphragm can be made of an elastomer. For most elastomers mechanical properties vary significantly even with modest temperature changes. With the spring **320,** a significant portion of the load applied to the needle **306** comes from the spring **320,** not the diaphragm **308.** Because the mechanical properties of spring steel (e.g., the material of the spring **320**) are far more constant for the same temperature change, adding the spring makes the system performance more consistent.

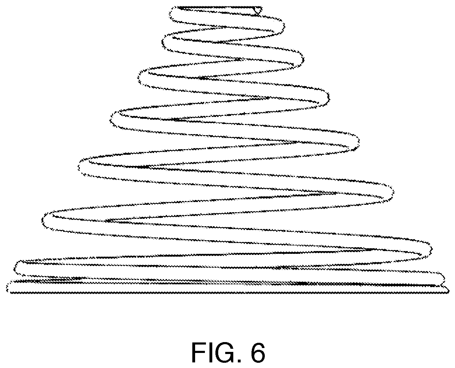

In an alternative embodiment, the disc spring **320** of FIG. 5 is replaced with a conical spring. A conical spring is similar to a conventional compression spring made of wire, but instead of being wound with a constant diameter, the diameter gets progressively smaller so that the spring has the shape of a cone, not a cylinder. See FIG. 6. When a conical spring is fully compressed, the coils nest within each other so the spring can become flat, only being as thick as the diameter of the wire it is wound from. Thus a fully compressed conical spring can fit in a similar form factor as the disc spring **320** of FIG. 5 and can serve the same function. The conical spring is cheaper, and it is easier to get a wide range of spring constants and operating deflections compared to the disc spring, so it is a feature of presently preferred embodiments.

The tip of needle **306** and/or the aperture it engages with can include an anti-microbial material.

## Claims

1. An apparatus for delivering a fluid to an eye of a patient, the apparatus comprising:
a fluid package comprising a reservoir (202) configured to hold a fluid, an aperture, and a needle (306) configured to seal the aperture when fluid is not being ejected through the aperture;
wherein the fluid package comprises a resilient diaphragm configured to provide a mechanical force to hold a tip of the needle in engagement with the aperture when fluid is not being ejected through the aperture;
wherein the resilient diaphragm is connected to the needle;
an actuator configured to eject the fluid through the aperture by providing a mechanical vibration at least to the needle; and **characterized in that** the apparatus comprises a venting arrangement configured to allow air to enter the reservoir as fluid is ejected from the reservoir wherein the venting arrangement is configured to equalize the pressure inside the reservoir (**202**) with ambient atmospheric pressure.

2. The apparatus of claim 1 wherein the venting arrangement includes an air inlet vent tube (**404**) that, in use, extends beyond liquid level of fluid (**302**) in the reservoir (**202**).

3. The apparatus of claim 2 wherein the vent tube (**404**) is connected to a venting outlet (**406**), which is open to the atmosphere.

4. The apparatus of claim 1, further comprising a particle filter configured to remove particles larger than 0.2 µm from air that enters the reservoir via the venting outlet.

5. The apparatus of claim 1, wherein the actuator includes an electromagnetic solenoid.

6. The apparatus of claim 1, wherein the actuator includes a coin vibration motor.

7. The apparatus of claim 1, wherein the fluid package comprises a resilient spring configured to provide an additional mechanical force to hold the tip of the needle in engagement with the aperture when fluid is not being ejected through the aperture.

8. The apparatus of claim 1, wherein the apparatus is configured to deliver a dose volume of 10 µl or less.

9. The apparatus of claim 1, wherein a diameter of the aperture is in a range between 200 µm and 600 µm.

10. The apparatus of claim 1, wherein a velocity of fluid ejected from the aperture is in a range from 1 m/s to 10 m/s.

11. The apparatus of claim 1, wherein an actuation pulse duration is 250 ms or less.

12. The apparatus of claim 1, wherein a repetition rate of actuation pulses is in a range from 10 Hz to 500 Hz.

13. The apparatus of claim 1, wherein the needle has a tip that engages with the aperture, and wherein the tip comprises an anti-microbial material.

14. The apparatus of claim 1, wherein the aperture comprises an anti-microbial material.

15. The apparatus of claim 1, wherein the aperture is in a front wall of the reservoir and wherein the diaphragm is in a rear wall of the reservoir.

## Patentansprüche

1. Einrichtung zum Abgeben eines Fluids an ein Auge eines Patienten, wobei die Einrichtung Folgendes umfasst:
eine Fluidpackung, die einen Behälter (202), der dazu konfiguriert ist, ein Fluid zu halten, eine Öffnung und eine Nadel (306) umfasst, die dazu konfiguriert ist, die Öffnung abzudichten, wenn kein Fluid durch die Öffnung ausgestoßen wird;
wobei die Fluidpackung eine elastische Membran umfasst, die dazu konfiguriert ist, eine mechanische Kraft bereitzustellen, um eine Spitze der Nadel in Eingriff mit der Öffnung zu halten, wenn kein Fluid durch die Öffnung ausgestoßen wird;
wobei die elastische Membran mit der Nadel verbunden ist;
einen Aktor, der dazu konfiguriert ist, das Fluid durch die Öffnung auszustoßen, indem eine mechanische Vibration mindestens an der Nadel bereitgestellt wird; und **dadurch gekennzeichnet, dass** die Einrichtung eine Belüftungsanordnung umfasst, die dazu konfiguriert ist, zu ermöglichen, dass Luft in den Behälter eintritt, wenn Fluid aus dem Behälter ausgestoßen wird, wobei die Belüftungsanordnung dazu konfiguriert ist, den Druck innerhalb des Behälters **(202)** mit Umgebungsatmosphärendruck auszugleichen.

2. Einrichtung nach Anspruch 1, wobei die Belüftungsanordnung ein Lufteinlassbelüftungsrohr **(404)** beinhaltet, das sich bei Verwendung über den Flüssigkeitspegel von Fluid **(302)** in dem Behälter **(202)** hinaus erstreckt.

3. Einrichtung nach Anspruch 2, wobei das Belüftungsrohr **(404)** mit einem Belüftungsauslass **(406)** verbunden ist, der gegenüber der Atmosphäre offen ist.

4. Einrichtung nach Anspruch 1, ferner umfassend einen Partikelfilter, der dazu konfiguriert ist, Partikel größer als 0,2 µm aus Luft zu entfernen, die über den Belüftungsauslass in den Behälter eintritt.

5. Einrichtung nach Anspruch 1, wobei der Aktor einen elektromagnetischen Elektromagneten beinhaltet.

6. Einrichtung nach Anspruch 1, wobei der Aktor einen Münzvibrationsmotor beinhaltet.

7. Einrichtung nach Anspruch 1, wobei die Fluidpackung eine elastische Feder umfasst, die dazu konfiguriert ist, eine zusätzliche mechanische Kraft bereitzustellen, um die Spitze der Nadel in Eingriff mit der Öffnung zu halten, wenn kein Fluid durch die Öffnung ausgestoßen wird.

8. Einrichtung nach Anspruch 1, wobei die Einrichtung dazu konfiguriert ist, ein Dosisvolumen von 10 µl oder weniger abzugeben.

9. Einrichtung nach Anspruch 1, wobei ein Durchmesser der Öffnung in einem Bereich zwischen 200 µm und 600 µm liegt.

10. Einrichtung nach Anspruch 1, wobei eine Geschwindigkeit des aus der Öffnung ausgestoßenen Fluids in einem Bereich von 1 m/s bis 10 m/s liegt.

11. Einrichtung nach Anspruch 1, wobei eine Betätigungsimpulsdauer 250 ms oder weniger beträgt.

12. Einrichtung nach Anspruch 1, wobei eine Wiederholungsrate von Betätigungsimpulsen in einem Bereich von 10 Hz bis 500 Hz ist.

13. Einrichtung nach Anspruch 1, wobei die Nadel eine Spitze aufweist, die in die Öffnung eingreift, und wobei die Spitze ein antimikrobielles Material umfasst.

14. Einrichtung nach Anspruch 1, wobei die Öffnung ein antimikrobielles Material umfasst.

15. Einrichtung nach Anspruch 1, wobei sich die Öffnung in einer vorderen Wand des Behälters befindet und wobei sich die Membran in einer hinteren Wand des Behälters befindet.

## Revendications

1. Appareil destiné à administrer un fluide dans l'œil d'un patient, l'appareil comprenant :
un emballage de fluide comprenant un réservoir (202) conçu pour contenir un fluide, une ouverture et une aiguille (306) conçue pour sceller l'ouverture lorsque le fluide n'est pas éjecté par l'ouverture ;
ledit emballage de fluide comprenant un diaphragme élastique conçu pour fournir une force mécanique afin de maintenir une pointe de l'aiguille en prise avec l'ouverture lorsque le fluide n'est pas éjecté par l'ouverture ;
ledit diaphragme élastique étant raccordé à l'aiguille ;
un actionneur conçu pour éjecter le fluide par l'ouverture en fournissant une vibration mécanique au moins à l'aiguille ; et **caractérisé en ce que** l'appareil comprend un agencement de ventilation conçu pour permettre à l'air d'entrer dans le réservoir lorsque le fluide est éjecté du réservoir, ledit agencement de ventilation étant conçu pour égaliser la pression à l'intérieur du réservoir **(202)** avec la pression atmosphérique ambiante.

2. Appareil de la revendication 1, ledit agencement de ventilation comprenant un tube d'évent d'entrée d'air **(404)** qui, lors de l'utilisation, s'étend au-delà du niveau de liquide du fluide **(302)** dans le réservoir **(202).**

3. Appareil de la revendication 2, ledit tube d'évent **(404)** étant relié à une sortie de ventilation **(406),** qui est ouverte sur l'atmosphère.

4. Appareil de la revendication 1, comprenant en outre un filtre à particules conçu pour éliminer les particules de plus de 0,2 µm de l'air qui pénètre dans le réservoir par l'intermédiaire de la sortie de ventilation.

5. Appareil de la revendication 1, ledit actionneur comprenant un solénoïde électromagnétique.

6. Appareil de la revendication 1, ledit actionneur comprenant un moteur de vibration de type pièce de monnaie.

7. Appareil de la revendication 1, ledit emballage de fluide comprenant un ressort élastique conçu pour fournir une force mécanique supplémentaire afin de maintenir la pointe de l'aiguille en prise avec l'ouverture lorsque le fluide n'est pas éjecté par l'ouverture.

8. Appareil de la revendication 1, ledit appareil étant conçu pour délivrer un volume de dose inférieur ou égal à 10 µl.

9. Appareil de la revendication 1, le diamètre de l'ouverture étant dans une plage comprise entre 200 µm et 600 µm.

10. Appareil de la revendication 1, une vitesse du fluide éjecté de l'ouverture étant comprise dans une plage allant de 1 m/s à 10 m/s.

11. Appareil de la revendication 1, une durée d'impulsion d'actionnement étant inférieure ou égale à 250 ms.

12. Appareil de la revendication 1, une fréquence de répétition des impulsions d'actionnement étant comprise dans une plage allant de 10 Hz à 500 Hz.

13. Appareil de la revendication 1, ladite aiguille comportant une pointe qui vient en prise avec l'ouverture, ladite pointe comprenant un matériau antimicrobien.

14. Appareil de la revendication 1, ladite ouverture comprenant un matériau antimicrobien.

15. Appareil de la revendication 1, ladite ouverture étant située dans une paroi avant du réservoir et ledit diaphragme étant situé dans une paroi arrière du réservoir.
